# EUROPEAN PATENT SPECIFICATION

(11) **EP 1 567 133 B1**
(45) Date of publication and mention of the grant of the patent: **02.05.2007**
(21) Application number: 03768857.9
(22) Date of filing: 07.11.2003
(51) Int. Cl.: A61K 9/20, A61K 31/7072

(54) **PHARMACEUTICAL ANTIVIRAL COMPOSITIONS**
ANTIVIRALE ZUSAMMENSETZUNGEN
PREPARATIONS PHARMACEUTIQUES ANTIVIRALES

(30) Priority: 08.11.2002 US 425182 P
(43) Date of publication of application: 31.08.2005
(73) Proprietor: GLAXO GROUP LIMITED, Greenford, Middlesex UB6 ONN (GB); SMITHKLINE BEECHAM CORPORATION, Philadelphia, PA 19101 (US)
(72) Inventor: GOODSON, Gary, Wayne, c/o GlaxoSmithKline, Research Triangle Park, NC 27709 (US); FLOYD, Alison, Green, c/o GlaxoSmithKline, Research Triangle Park, NC 27709 (US); NAVY, Cecilia, Carpenter, c/o GlaxoSmithKline, Research Triangle Park, NC 27709 (US)
(74) Representative: Gladwin, Amanda Rachel
(86) International application number: PCT/US2003/035830
(87) International publication number: WO 2004/043433

(56) References cited:
- WO-A-98/18477
- US-A- 4 917 900
- US-A- 5 681 581
- US-B1- 6 177 435

## Description

The present Invention relates to pharmaceutical compositions combining the agents 3'-azido-3'-deoxythymidine and (2R,cis)-4-amino-1-(2-hydroxymethyl-1,3-oxathiolan-5-yl)-(1H)-pyrimidin-2-one into a single form, useful in the treatment of Human Immunodeficiency Virus (HIV) infections.

### BACKGROUND OF THE INVENTION

Retrovir^{®} (zidovudine), also known as 3'-azido-3'-deoxythymidine, is approved for the treatment of HIV infections in combination with other antiretroviral agents. It is also indicated for the prevention of maternal-fetal HIV transmission as part of a regiment that includes oral zidovudine beginning between 14 and 34 weeks of gestation, intravenous zidovudine during labor, and administration of zidovudine syrup to the neonate after birth.

Therapy with Retrovir^{®} has been shown to prolong survival and decrease the incidence of opportunistic infections in patients with advanced HIV disease and to delay disease progression in asymptomatic HIV-infected patients.

The recommended oral dose of Retrovir^{®} is 600 mg per day in divided doses in combination with other antiretroviral agents. The recommended dose in pediatric patients 6 weeks to 12 years of age is 160 mg/m² every 8 hours in combination with other antiretroviral agents.

Zidovudine is rapidly absorbed and distributed, with peak serum concentrations occurring within 0.5 and 1.5 hours. Elimination is rapid with terminal half-life of approximately 1.5 - 2 hours. It is renally eliminated by hepatic metabolism.

(2R,cis)-4-Amino-1-(2-hydroxymethyl-1,3-oxathiolan-5-yl)-(1H)-pyrimidin-2-one, (also known as lamivudine, EPIVIR^{®}, 3TC^{®}, -cis-1-[2-(hydroxymethyl)-1,3-oxathiolan-5-yl]cytosine, (-)2',3'-dideoxy, 3'-thiacytidine) has proven antiviral activity against human immunodeficiency virus (HIV) and other viruses such as hepatitis B. (2R,cis)-4-Amino-1-(2-hydroxymethyl-1,3-oxathiolan-5-yl)-(1H)-pyrimidin-2-one and its use against HIV are described in EP 0382526 and WO91/17159. Crystalline forms of (2R,cis)-4-amino-1-(2-hydroxymethyl-1,3-oxathiolan-5-yl)-(1H)-pyrimidin-2-one are described in WO92/21676. Combinations of (2R,cis)-4-amino-1-(2-hydroxymethyl-1,3-oxathiolan-5-yl)-(1H)-pyrimidin-2-one with other reverse transcriptase inhibitors are described in WO92/20344.

The success of modem multiple-drug treatments for HIV often requires strict compliance with a complex treatment regimen that can require the administration of many different drugs per day, administered at precisely timed intervals with careful attention to diet. Patient non-compliance is a well known problem accompanying such complex treatment regimens. Patient non-compliance is a critical problem in the treatment of HIV because such non-compliance may lead to the emergence of multiple-drug resistant strains of HIV.

The present invention addresses the issue of non-compliance by formulating multiple active ingredients, 3'-azido-3'-deoxythymidine and (2R,cis)-4-amino-1-(2-hydroxymethyl-1,3-oxathiolan-5-yl)-(1H)-pyrimidin-2-one, into a single tablet for once daily administration. However, simply combining the two drugs, at therapeutic doses for once per day administration, into a single tablet would result in a tablet size too large to swallow without difficulty. Furthermore, the greater the amount of drug in the formulation, the more excipients are needed in order to compress the mixture into a tablet. Increased amounts of some excipients can have adverse effects on tablet properties and can lead to problems of, for example, dissolution, content uniformity, hardness, and segregation.

Moreover, it was thought that the short half-life of 3'-azido-3'-deoxythymidine in the blood and the drug's preferential absorption in the upper part of the gastrointestinal tract would preclude once daily dosing.

We have discovered a pharmaceutical composition comprising zidovudine and lamivudine that matches the body's absorption rate of zidovudine with the area of the gastrointestinal tract, thereby controlling blood levels of zidovudine and reducing potential side effects. The compositions of the present invention provide for once daily dosing, thereby addressing the problem of patient non-compliance and pill burden.

### SUMMARY OF THE INVENTION

It is a feature of the present invention to provide pharmaceutical compositions comprising the active ingredients 3'-azido-3'-deoxythymidine (zidovudine) and (2R,cis)-4-amino-1-(2-hydroxymethyl-1,3-oxathiolan-5-yl)-(1H)-pyrimidin-2-one (lamivudine), or pharmaceutically acceptable derivatives thereof, in the form of a tablet that provides for differential release of the active ingredients.

A further feature of the present invention is to provide a method for using these pharmaceutical compositions.

### DETAILED DESCRIPTION OF THE INVENTION

The present invention provides pharmaceutical compositions comprising the active ingredients 3'-azido-3'-deoxythymidine and (2R,cis)-4-amino-1-(2-hydroxymethyl-1,3-oxathiolan-5-yl)-(1H)-pyrimidin-2-one, or pharmaceutically acceptable derivatives thereof, in the form of a tablet that provides for differential release of the active ingredients.

A further feature of the present invention is to provide a method for using these pharmaceutical compositions.

The present invention features a pharmaceutical composition, comprising one layer containing a safe and therapeutically effective amount of zidovudine and a safe and therapeutically effective amount lamivudine; and a second layer containing a safe and therapeutically effective amount of zidovudine or a pharmaceutically acceptable derivative thereof and a pharmaceutically acceptable carrier.

The phrase "safe and therapeutically effective amount," as used herein, means a sufficient amount of a drug, compound, composition, product or pharmaceutical agent to abate or reverse or treat a malady in a human or other mammal without severely harming the tissues of the mammal to which the drug or pharmaceutical agent is administered.

The phrase "pharmaceutically acceptable derivative," as used herein, means any pharmaceutically acceptable salt, solvate, ester, or salt of such ester, or any other compound which, upon administration to the recipient, is capable of providing (directly or indirectly) the intended active ingredient or any active metabolite or residue thereof.

The phrase "pharmaceutically acceptable derivative of zidovudine" as used herein, means any pharmaceutically acceptable salt, solvate, ester, or salt of such ester, of zidovudine, or any other compound which, upon administration to the recipient, is capable of providing (directly or indirectly) zidovudine or any antivirally active metabolite or residue thereof.

The phrase "pharmaceutically acceptable derivative of lamivudine" as used herein, means any pharmaceutically acceptable salt, solvate, ester, or salt of such ester, of lamivudine, or any other compound which, upon administration to the recipient, is capable of providing (directly or indirectly) lamivudine or any antivirally active metabolite or residue thereof. Examples of lamivudine salts are hydrochloride and mesylate.

Preferably, (2R,cis)-4-amino-1-(2-hydroxymethyl-1,3-oxathiolan-5-yl)-(1H)-pyrimidin-2-one is provided substantially free of the corresponding (+)-enantiomer. "Substantially free" as used herein, means that there is less than about 10% w/w of the (+)-enantiomer present compared with the amount of (2R,cis)-4-amino-1-(2-hydroxymethyl-1,3-oxathiolan-5-yl)-(1H)-pyrimidin-2-one. Preferably there is less than about 5% w/w of the (+)-enantiomer present compared with the amount of (2R,cis)-4-amino-1-(2-hydroxymethyl-1,3-oxathiolan-5-yl)-(1H)-pyrimidin-2-one.

The present invention features pharmaceutical compositions comprising a controlled release formulation of 3'-azido-3'-deoxythymidine or a pharmaceutically acceptable derivative thereof, and an immediate release formulation of 3'-azido-3'-deoxythymidine or a pharmaceutically acceptable derivative thereof and (2R,cis)-4-amino-1-(2-hydroxymethyl-1,3-oxathiolan-5-yl)-(1H)-pyrimidin-2-one or a pharmaceutically acceptable derivative thereof. Advantageously, pharmaceutical compositions comprise one layer containing a controlled release formulation of 3'-azido-3'-deoxythymidine or a pharmaceutically acceptable derivative thereof; and a second layer containing an immediate release formulation of (2R,cis)-4-amino-1-(2-hydroxymethyl-1,3-oxathiolan-5-yl)-(1H)-pyrimidin-2-one or a pharmaceutically acceptable derivative thereof and 3'-azido-3'-deoxythymidine or a pharmaceutically acceptable derivative thereof. Preferably, the compositions comprise 3'-azido-3'-deoxythymidine and (2R,cis)-4-amino-1-(2-hydroxymethyl-1,3-oxathiolan-5-yl)-(1H)-pyrimidin-2-one.

The compositions of the present invention provide for an immediate release of zidovudine and lamivudine for the initial release period, thereby ensuring safety in administering the drugs followed by a controlled release of zidovudine in the small intestines to maximize absorption in that portion of the gastrointestinal tract, because the colonic absorption of zidovudine is very low. Therefore, in order to achieve therapeutic levels of drug, zidovudine advantageously is released from the composition within approximately 3 - 6 hours, most advantageously within 4- 5 hours. The immediate release formulation in combination with a controlled release formulation according to the present invention may provide for release of drug to achieve therapeutic levels over the longest time period of absorption. The total release of drug may be over the range of time 0 to 3 - 6 hours, preferably 0 to 4 - 5 hours. Compositions according to the present invention may provide release of drug during the early and later hours of this range.

An embodiment of the present invention is a bilayer tablet. That is, one layer may contain a controlled release formulation of zidovudine and a second layer may contain an immediate release formulation of zidovudine and lamivudine. Other embodiments include a tablet within a tablet, multiple layered tablets (i.e. more than two layers), controlled released beads containing drug, osmotic agents as in osmotic pumps, and rate controlling film coatings. The immediate release part of the composition may contain the active ingredients in amorphous form, crystalline form, or a mixture thereof, advantageously in a finely grounded or micronized crystalline form. The release rate may be influenced by the addition of auxiliary ingredients with good water solubility and by alteration of the particle size distribution of the active compound.

In general 50 - 100 % of zidovudine and 50 - 100% of lamivudine in the immediate release part of the composition may be released in 0.5 - 2 hours or less, advantageously in about 5 - 30 minutes or less. Dissolution testing of lamivudine and zidovudine may be performed using USP Apparatus II (paddles) at 75 RPM with 0.1N HCl dissolution medium, previously equilibrated at 37 °C.

The immediate release part of the composition may contain excipients that may help increase the solubility and permeability in the small intestine and in the large intestine, thereby increasing the bioavailability of this portion of the composition. In general, examples of such excipients may include pH modifying agents that modify the solubility, hygroscopic or deliquescent agents to increase water availability, effervescent ingredients to produce CO₂ and water, hydrates to generate water for dissolution, and/or co-solvents to, for example, enhance solubility.

If an amorphous active compound is employed in the immediate release part of the composition, it may be dissolved in water or in an organic solvent, or mixture thereof, together with one or more polymers using techniques and polymers known to those skilled in the art.

When using crystalline active pharmaceutical ingredients in the immediate release part of the composition, it may be desirable to add readily water-soluble auxiliary ingredients, for example, lactose. The release rate may be accelerated by the use of disintegrants, for example, crosslinked polyvinylpyrrolidone (PVP), or surface-active substances, for example, sodium lauryl sulfate.

The preparation of the immediate release part of the composition may be performed by methods known to those skilled in the art. In general, the desired ingredients such as active ingredient, microcrystalline cellulose, magnesium stearate, may be granulated in a granulator or roller compactor, optionally with water and/or water containing a binder, into a dry powder blend or wet granulation. The dry blend or wet granulation may be milled and dried one or several times until it is capable of being seived or compressed. Alternatively, a direct compression, dry blend process may be used.

The controlled release part of the composition may contain at lease one controlled release agent selected from the group consisting of hydrophilic gel-forming polymers, water insoluble permeable polymers, and mixtures thereof. The controlled release part may contain from about 10 % to about 70% by weight of such polymers. Suitable polymers include, but are not limited to, modified starch or cellulose-like substances, for example methylcellulose, ethylcellulose, hydroxypropylmethylcellulose, hydroxypropylethylcellulose, hydroxypropylcellulose, and sodium carboxymethylcellulose. Advantageously, hydroxypropylmethylcellulose may be used.

The present invention features a pharmaceutical composition comprising a controlled release formulation of 3'-azido-3'-deoxythymidine or a pharmaceutically acceptable derivative thereof, and an immediate release formulation of 3'-azido-3'-deoxythymidine or a pharmaceutically acceptable derivative thereof and (2R,cis)-4-amino-1-(2-hydroxymethyl-1,3-oxathiolan-5-yl)-(1H)-pyrimidin-2-one or a pharmaceutically acceptable derivative thereof wherein the controlled release formulation comprises a mixture of polymers. Polymers may be selected for their viscosity and hydrophilic and hydrophobic properties in order to achieve a release rate of zidovudine of approximately 4 to 6 hours, preferably 4 to 5 hours. Advantageously, the polymers are present in an amount from about 10% to about 15% w/w of the controlled release part of the composition. Polymers are present in amounts that allow for controlled release of drug as defined above while maintaining ease of processing, matrix integrity, and reasonable tablet size for administration to patients. Advantageously, mixtures of hydroxypropylmethylcellulose of different viscosities may be used.

The preparation of the controlled release part of the formulation may be performed by any method know to those skilled in the art. Such methods are disclosed, for example, in Sheskey P.J., et al., "Use of Roller Compaction in the Preparation of Controlled-Release Hydrophilic Matrix Tablets Containing Methylcellulose and Hydroxypropyl Methylcellulose Polymers", Pharmaceutical Technology, September 1994 and Sheskey P.J., et al., "Comparison of Low-Shear and High-Shear Wet Granulation Techniques and the Influence of Percent Water Addition in the Preparation of a Controlled-Release Matrix Tablet Containing HPMC and a High-Dose, Highly Water-Soluble Drug", Pharmaceutical Technology, March 1996.

In general, in order to prepare the controlled release part of the composition, particles of active pharmaceutical ingredient, for example zidovudine, are blended with a controlled release agent, for example hydroxypropylmethylcellulose (HPMC) and optionally with microcrystalline cellulose. Optionally, a binder, for example polyvinylpyrollidone or hydroxypropylcellulose, is dissolved in water to form a granulating solution. Thereafter, the granulating solution may be sprayed onto the blended powders, which may then be dried. Optionally, a lubricant, for example magnesium stearate, may be added and blended with the previously blended powders having the granulating solution dried thereon. Other suitable fillers include, but are not limited to, lactose, starch, and dibasic calcium phosphate. A stabilizer or binder may be absent from both the immediate and controlled release parts of the composition or a stabilizer or binder may be used in either or both of the immediate release and controlled release parts of the composition.

The compositions of the present invention may contain additional ingredients. Such ingredients include, but are not limited to, binders, coating materials, osmotic agents, diluents, excipients, lubricants, fillers, glidants, plasticizers, disintegrants, and the like.

Binders may include, but are not limited to, modified starch, gelatin, and other polymers. Suitable binders may include corn starch, potato starch, or other starches, gelatin, natural and synthetic gums, for example, acacia, sodium alginate, alginic acid, other alignates, powdered tragacanth, guar gum, cellulose and its derivatives, for example, ethylcellulose, cellulose acetate, carboxymethyl cellulose calcium, sodium carboxymethyl cellulose, pre-gelatinized starch, microcrysltalline cellulose, and mixtures thereof.

Tablets may be film coated on the outside or film coated to separate immediate release and controlled release parts of the composition. Enteric, identification, appearance, and taste-masking coatings may be employed as well as coatings that enhance immediate release or controlled release capability. A coating may be applied by compression or spray drying. Spray drying the outer coating of a tablet may have the added advantage of producing a smaller, easier to swallow tablet.

Typical film coats may include, but are not limited to, cellulose ether, ethylcellulose, cellulose ester, for example, cellulose acetate, polyvinylalcohol. Other coating materials may include, but are not limited to, water-soluble polymers, for example, polyethylene glycol, or polysaccharides such as sorbate derivatives may be used a film-forming coating and may include cellulose ether, such as ethylcellulose.

Osmotic agents may include, but are not limited to, sodium chloride, potassium chloride, magnesium sulfate, magnesium chloride, sodium sulfate, lithium sulfate, urea, inositol, sucrose, lactose, glucose, sorbitol, fructose, mannitol, dextrose, magnesium succinate, potassium acid phosphate and the like.

Other excipients may include, but are not limited to, acrylic acid derivatives, waxes such as carnauba wax, water insoluble-water semipermeable polymers such as methylcellulose and microcrystalline cellulose, water insoluble-water pH dependent polymers, such as acrylic acid-acrylic ester combinations and methacrylic acid-methacrylic ester combinations, and the like.

Lubricants may include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil, for example peanut oil, cottonseed oil, sunflower oil, sesame oil, olive oil, corn oil, and soybean oil, zinc stearate, ethyl oleate, ethyl laureate, agar, syloid silica gel, coagulated aerosol of synthetic silica, Cab-o-sil^{®} (Cabot Co.), or mixtures thereof.

Fillers may include, but are not limited to, talc, calcium carbonate, microcrystalline cellulose, powdered cellulose, dextrose, kaolin, mannitol, silicic acid, sorbitol, lactose, microcrystalline cellulose, starch, pre-gelatinized starch, and mixtures thereof. The binder/filler in pharmaceutical compositions of the present invention may be present in an amount from about 5 % to 65 % w/w of the composition.

Glidants may include, but are not limited to, silicon dioxide, for example, colloidal silicon dioxide.

Plasticizers may include, but are not limited to, citrate ester, oil for example castor oil, polyalkyleneglycol for example polyethylene glycol, phthalate esters for example diethyl phthalate, citric acid esters for example triethyl citrate.

Disintegrants may include, but are not limit to, agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, clays, other algins, other celluloses, gums or mixtures thereof.

Another feature of the present invention is to simplify treatment regimens for HIV and other viruses with the goal of enhancing patient compliance by providing a simplified dosage form containing pharmaceutically acceptable amounts of 3'-azido-3'-deoxythymidine and (2R,cis)-4-amino-1-(2-hydroxymethyl-1,3-oxathiolan-5-yl)-(1H)-pyrimidin-2-one or pharmaceutically acceptable derivatives thereof.

The present invention also features a method for treating, reversing, reducing or inhibiting retroviral infections in particular HIV infections, in a mammal, in particular a human, which method comprises administering to said mammal a safe and effective amount of a composition according to the invention.

It will be appreciated by those skilled in the art that reference herein to "treatment" extends to both the prophylaxis and the treatment of an established malady, infection or its symptoms.

The compositions of the present invention may include from 0 to about 5% magnesium stearate; from about 0.05 to about 10% glidant; from 0 to about 15% sodium starch glycollate; and from about 10 to about 60% microcrystalline cellulose.

The pharmaceutical compositions of the present invention may optionally contain silicon dioxide (SiO₂), also referred to as colloidal silica, fumed silicon dioxide, fumed silica, light anhydrous silicic acid, silicic anhydride, AEROSIL^{™} or CAB-O-SIL^{™}; asbestos free talc, sodium aluminosilicate, calcium silicate, powdered cellulose, microcrystalline cellulose, corn starch, sodium benzoate, calcium carbonate, magnesium carbonate, metallic stearates, calcium stearate, magnesium stearate, zinc stearate, stearowet C, starch, starch 1500, magnesium lauryl sulfate, magnesium oxide, colloidal silicon dioxide in combination with microcrystalline cellulose or ProSolve^{™}.

Methods for the preparation of lamivudine are described in, inter alia WO 91/17159, WO 92/21676, WO 92/20669, WO 95/29174, incorporated herein by reference.

Zidovudine can be prepared, for example, as described in U.S. Patent 4,724,232, incorporated herein by reference hereto. Zidovudine can also be obtained from Aldrich Chemical Co., Milwaukee, WI 53233, USA.

The pharmaceutical compositions of the invention are advantageously presented as pharmaceutical compositions suitable for oral administration. Such compositions may conveniently be presented as discrete units such as tablets, caplets, capsules, or any other form suitable for oral administration and compatible with the compositions of the present invention, each containing a predetermined amount of the active ingredients. A particularly suitable composition may be prepared from direct compression or granulation processes. Such compositions may contain safe and effective amounts of conventional excipients such as binding agents, fillers, lubricants, or disintegrants. The tablets may also be coated according to any method known to persons skilled in the art that would not interfere with the tablets' release properties, or the other physical or chemical characteristics of the present Invention. Tablet coating is further described and delineated by Remington, The Science & Practice of Pharmacy 19th ed. 1995 incorporated herein by reference. The compositions may also include a safe and effective amount of other active ingredients, such as antimicrobial agents or preservatives

These compositions of the present invention are suitable for administration to humans or other mammals particularly via an oral route of administration. However, other routes as utilised by medical practitioners and others skilled in the art of pharmaceutical dosage administration such as pharmacists and nurses are not foreclosed.

It will be appreciated by those skilled in the art that the amount of active ingredients required for use in treatment will vary according to a variety of factors, including the nature of the condition being treated and the age and condition of the patient, and will ultimately be at the discretion of the attending physician, veterinarian or health care practitioner.

The current recommended oral dose of Epivir^{®} (lamivudine) for adults and adolescents is 150 mg twice daily administered in combination with other antiretroviral agents. For adults with low body weights (less than 50 kg or 110 lb.) the current recommended oral dose of lamivudine is 2mg/kg twice daily administered in combination with other antiretroviral agents. The recommended oral dose of lamivudine in paediatric patients 3 months to 12 years of age is 4mg/kg twice daily, up to a maximum of 150 mg twice daily administered in combination with other antiretroviral agents.

The recommended oral dose of Retrovir^{®} (zidovudine) is 600 mg per day in divided doses in combination with other antiretroviral agents. The recommended dose in pediatric patients 6 weeks to 12 years of age is 160 mg/m² every 8 hours in combination with other antiretroviral agents.

Compositions of the present invention may contain 20 - 60 % w/w zidovudine and 20 - 60% w/w lamivudine in one layer and 40 - 80% w/w zidovudine in the other layer. Advantageously, the compositions may contain 40% w/w zidovudine and 40% w/w lamivudine in one layer and 60% zidovudine in the other layer.

Compositions of the present invention enable patients greater freedom from multiple dosage medication regimens and ease the needed diligence required in remembering complex daily dosing times and schedules. By combining zidovudine and lamivudine into a single dosage form with differential release rates, the desired daily doses may be presented in a single dose. The compositions of the present invention are particularly suitable for administration as a single dose once daily. Advantageously, the compositions of the present invention may be administered once daily.

The compositions of the present invention conveniently allow administration of two separate compounds in unit dosage form containing, for example, from about 100 mg to about 750 mg of zidovudine, particularly from about 450 mg to about 600 mg of zidovudine, and most particularly about 600 mg of zidovudine, and from about 50 mg to about 450 mg of lamivudine, particularly from about 200 to about 400 mg of lamivudine and most particularly 300 mg of lamivudine per unit dosage form. The composition of the present invention may be used in combination with other pharmaceutical formulations as a component of a multiple drug treatment regimen.

Compositions of the present invention may also be packaged as articles of manufacture comprising a safe and therapeutically effective amount of zidovudine, or a pharmaceutically acceptable derivative thereof; and a safe and therapeutically effective amount of lamivudine, or a pharmaceutically acceptable derivative thereof.

Any of the various methods known by persons skilled in the art for packaging tablets, caplets, or other solid dosage forms suitable for oral administration, that will not degrade the components of the present invention, are suitable for use in packaging. Tablets, caplets, or other solid dosage forms suitable for oral administration, may be packaged and contained in various packaging materials particularly glass and plastic bottles and also including unit dose blister packaging. The packaging material may also have labelling and information related to the pharmaceutical composition printed thereon. Additionally, an article of manufacture may contain a brochure, report, notice, pamphlet, or leaflet containing product information. This form of pharmaceutical information is referred to in the pharmaceutical industry as a "package insert." A package insert may be attached to or included with a pharmaceutical article of manufacture. The package insert and any article of manufacture labelling provides information relating to the pharmaceutical composition. The information and labelling provides various forms of information utilised by health-care professionals and patients, describing the composition, its dosage and various other parameters required by regulatory agencies such as the United States Food and Drug Agencies.

The compositions of the present invention can be formulated using methods and techniques suitable for the compositions physical and chemical characteristics and that are commonly employed by persons skilled in the art in preparing oral dosage forms utilising direct compression or granulation processes. Remington, The Science & Practice of Pharmacy, p. 1615-1623, 1625-1648, and other applicable sections, 19th ed. (1995).

Compositions of the present invention in their method aspect are administered to a human or other mammal in a safe and effective amount as described herein. These safe and effective amounts will vary according to the type and size of mammal being treated and the desired results of the treatment.

### EXAMPLES

The following examples further describe and demonstrate particular embodiments within the scope of the present Invention.

### Example 1

### Manufacturing Formula

### Immediate Release Material

| **Material** | **Function** | **Unit Qty (mg/tab)** | **% w/w** | **Unit Qty (kg/batch)** |
|---|---|---|---|---|
| **Immediate Release Layer** | | | | |
| Zidovudine | active | 300 | 35.29 | 1.41 |
| Lamivudine | active | 300 | 35.29 | 1.41 |
| Avicel PH 102 | Diluent/binder | 215.65 | 25.37 | 1.01 |
| Sodium starch glycolate | disintegrant | 25.50 | 3.0 | 0.12 |
| Colloidal silicon dioxide | glidant | 2.55 | 0.3 | 0.012 |
| Magnesium stearate | lubricant | 6.38 | 0.75 | 0.03 |
| Total | | ***850*** | ***100*** | 4.0 |

### Weighing and Blending

1. The appropriate amounts (see table above) of zidovudine, lamivudine, avicel, sodium starch glycolate, and colloidal silicon dioxide were weighed and recorded in the table above.
2. Colloidal silicon dioxide and avicel were mixed together for screening purposes.
3. The materials were screened in the following order: zidovudine, lamivudine, avicel and colloidal silicon dioxide mixture, and sodium starch glycolate.
4. The sieved ingredients from step 3 above were added to a 16 quart V-shell blender.
5. The ingredients were blended for 15 minutes.
6. The appropriate amount of magnesium stearate was weighed and the weight recorded in table above.
7. The magnesium stearate was screened.
8. The magnesium stearate was added to the blended material and lubricated for 2 minutes.

### Manufacturing Formula

### Controlled Release Material

| **Material** | **Function** | **Unit Qty (mg/tab)** | **% w/w** | **Unit Qty (kg/batch)** |
|---|---|---|---|---|
| **Controlled Release Layer** | | | | |
| Zidovudine | active | 300 | 60.0 | 3.0 |
| HPMC K100LVP | Control release | 25 | 5 | 0.25 |
| HPMC E4MP | Control release | 25 | 5 | 0.25 |
| Avicel PH 102 | Diluent/Binder | 143.75 | 28.75 | 1.4375 |
| Colloidal Silicon Dioxide | glidant | 2.5 | 0.5 | 0.025 |
| Magnesium stearate | lubricant | 3.75 | 0.75 | 0.0375 |
| Total | | ***500*** | ***100*** | ***5.00*** |

### Weighing and Blending

9. The appropriate amounts (see table above)of zidovudine, K100LVP, E4M, avicel, and colloidal silicon dioxide were weighed and recorded in table above.
10. Colloidal silicon dioxide and the avicel were mixed together for screening purposes.
11. The materials were screened in the following order: zidovudine, K100LVP, E4M, and avicel and colloidal silicon dioxide mixture.
12. The sieved ingredients from step 10 above were added to a 16 quart V-shell blender.
13. The ingredients were blended for 15 minutes.
14. The appropriate amount of magnesium stearate was weighed and the weight was recorded in table above.
15. The magnesium stearate was screened.
16. The magnesium stearate was added to the blended material and blended for 2 minutes.

### Compression:

The immediate release tablet blend and the controlled release tablet blend were compressed using a suitable bilayer tablet press or multilayer tablet press to yield final tablets.

Alternatively, either the immediate release layer or controlled release layer can be processed by wet granulation where the drug substances are in the blend of excipients or dissolved or suspended in the binder solution or granulating solvent. Alternatively, either the immediate release layer or controlled release layer can be processed by roller compaction where the excipients and drug substances are compacted under various pressures to produce granules suitable for tablet compression. Compression can be achieved on a standard tablet press or a bilayer tablet press.

## Claims

1. A pharmaceutical composition comprising:
i) a controlled release formulation of 3'-azido-3'-deoxythymidine or any pharmaceutically acceptable salt, solvate, ester, or salt of such ester, thereof; and
ii) an immediate release formulation of 3'-azido-3'-deoxythymidine or any pharmaceutically acceptable salt, solvate, ester, or salt of such ester, thereof and (2R,cis)-4-amino-1-(2-hydroxymethyl-1,3-oxathiolan-5-yl)-(1H)-pyrimidin-2-one or any pharmaceutically acceptable salt, solvate, ester, or salt of such ester, thereof.

2. A pharmaceutical composition comprising:
i) one layer containing a controlled release formulation of 3'-azido-3'-deoxythymidine or any pharmaceutically acceptable salt, solvate, ester, or salt of such ester, thereof; and
ii) a second layer containing an immediate release formulation of (2R,cis)-4-amino-1-(2-hydroxymethyl-1,3-oxathiolan-5-yl)-(1H)-pyrimidin-2-one or any pharmaceutically acceptable salt, solvate, ester, or salt of such ester, thereof and 3'-azido-3'-deoxythymidine or any pharmaceutically acceptable salt, solvate, ester, or salt of such ester, thereof.

3. A pharmaceutical composition according to claim 1 or 2 wherein the controlled release formulation releases 3'-azido-3'-deoxythymidine within 3 - 6 hours.

4. A pharmaceutical composition according to any of claims 1 - 3 wherein the controlled release formulation comprises a mixture of polymers.

5. A pharmaceutical composition according to claim 4 wherein the polymers are hydroxypropylmethylcellulose.

6. A pharmaceutical composition according any of claims 1 - 5 wherein the amount of (2R,cis)-4-amino-1-(2-hydroxymethyl-1,3-oxathiolan-5-yl)-(1H)-pyrimidin-2-one is from 150 to 450 mg per unit dosage form.

7. A pharmaceutical composition according any of claims 1 - 5 wherein the amount of 3'-azido-3'-deoxythymidine is from 100 to 750 mg per unit dosage form.

8. The pharmaceutical composition according to any of claims 1 - 6 wherein (2R,cis)-4-amino-1-(2-hydroxymethyl-1,3-oxathiolan-5-yl)-(1H)-pyrimidin-2-one is provided substantially free of the corresponding (+)-enantiomer.

9. The pharmaceutical composition according to any of claims 1 - 6 wherein (2R,cis)-4-amino-1-(2-hydroxymethyl-1,3-oxathiolan-5-yl)-(1H)-pyrimidin-2-one is provided such that the corresponding (+)-enantiomer is present in an amount of not more than about 5% w/w of the amount of (2R,cis)-4-amino-1-(2-hydroxymethyl-1,3-oxathiolan-5-yl)-(1H)-pyrimidin-2-one.

10. A pharmaceutical composition according to any of claims 1 - 9 for once daily administration.

11. A pharmaceutical composition according to any of claims 1 - 9 in the form of a bilayer tablet or multilayer tablet.

12. A pharmaceutical composition according to any of claims 1 - 9 in the form of a bilayer tablet.

13. A pharmaceutical composition according to any of claims 1 - 9 in the form of a tablet within a tablet.

14. Use of 3'-azido-3'-deoxythymidine or any pharmaceutically acceptable salt, solvate, ester or salt of such ester, thereof and (2R,cis)-4-amino-1-(2-hydroxymethyl-1,3-oxathiolan-5-yl)-(1H)-pyrimidin-2-one or any pharmaceutically acceptable salt, solvate, ester or salt of such ester, thereof for the preparation of a pharmaceutical composition according to any of claims 1-13 for the treatment or prevention of a HIV infection in a mammal.

## Patentansprüche

1. Pharmazeutische Zusammensetzung, die folgendes umfaßt:
i) eine Formulierung von 3'-Azido-3'-desoxythymidin oder einem pharmazeutisch akzeptablen Salz, Solvat, Ester oder Salz eines solchen Esters davon mit kontrollierter Freisetzung; und
ii) eine Formulierung von 3'-Azido-3'-desoxythymidin oder einem pharmazeutisch akzeptablen Salz, Solvat, Ester oder Salz eines solchen Esters davon und von (2R,cis)-4-Amino-1-(2-hydroxymethyl-1,3-oxathiolan-5-yl)-(1H)-pyrimidin-2-on oder einem pharmazeutisch akzeptablen Salz, Solvat, Ester oder Salz eines solchen Esters davon mit unmittelbarer Freisetzung.

2. Pharmazeutische Zusammensetzung, die folgendes umfaßt:
i) eine Schicht, die eine Formulierung von 3'-Azido-3'-desoxythymidin oder einem pharmazeutisch akzeptablen Salz, Solvat, Ester oder Salz eines solchen Ester davon mit kontrollierter Freisetzung enthält; und
ii) eine zweite Schicht, die eine Formulierung von (2R,cis)-4-Amino-1-(2-hydroxymethyl-1,3-oxathiolan-5-yl)-(1H)-pyrimidin-2-on oder einem pharmazeutisch akzeptablen Salz, Solvat, Ester oder Salz eines solchen Esters davon und von 3'-Azido-3'-desoxythymidin oder einem pharmazeutisch akzeptablen Salz, Solvat, Ester oder Salz eines solchen Esters davon mit unmittelbarer Freisetzung enthält.

3. Pharmazeutische Zusammensetzung gemäß Anspruch 1 oder 2, worin die Formulierung mit kontrollierter Freisetzung 3'-Azido-3'-desoxythymidin innerhalb von 3-6 Stunden freisetzt.

4. Pharmazeutische Zusammensetzung gemäß einem der Ansprüche 1 bis 3, worin die Formulierung mit kontrollierter Freisetzung eine Mischung von Polymeren umfaßt.

5. Pharmazeutische Zusammensetzung gemäß Anspruch 4, worin die Polymere Hydroxypropylmethylcellulose sind.

6. Pharmazeutische Zusammensetzung gemäß einem der Ansprüche 1 bis 5, worin die Menge von (2R,cis)-4-Amino-1-(2-hydroxymethyl-1,3-oxathiolan-5-yl)-(1H)-pyrimidin-2-on 150 bis 450 mg pro Einheitsarzneiform beträgt.

7. Pharmazeutische Zusammensetzung gemäß einem der Ansprüche 1 bis 5, worin die Menge von 3'-Azido-3'-desoxythymidin 100 bis 750 mg pro Einheitsarzneiform beträgt.

8. Pharmazeutische Zusammensetzung gemäß einem der Ansprüche 1 bis 6, worin (2R,cis)-4-Amino-1-(2-hydroxymethyl-1,3-oxathiolan-5-yl)-(1H)-pyrimidin-2-on im wesentlichen frei vom entsprechenden (+)-Enantiomer bereitgestellt wird.

9. Pharmazeutische Zusammensetzung gemäß einem der Ansprüche 1 bis 6, worin (2R,cis)-4-Amino-1-(2-hydroxymethyl-1,3-oxathiolan-5-yl)-(1H)-pyrimidin-2-on derart bereitgestellt wird, daß das entsprechende (+)-Enantiomer in einer Menge von nicht mehr als ca. 5 % G/G der Menge von (2R,cis)-4-Amino-1-(2-hydroxymethyl-1,3-oxathiolan-5-yl)-(1H)-pyrimidin-2-on vorhanden ist.

10. Pharmazeutische Zusammensetzung gemäß einem der Ansprüche 1 bis 9 zur einmal täglichen Verabreichung.

11. Pharmazeutische Zusammensetzung gemäß einem der Ansprüche 1 bis 9 in Form einer Zweischichttablette oder Mehrschichttablette.

12. Pharmazeutische Zusammensetzung gemäß einem der Ansprüche 1 bis 9 in Form einer Zweischichttablette.

13. Pharmazeutische Zusammensetzung gemäß einem der Ansprüche 1 bis 9 in Form einer Tablette innerhalb einer Tablette.

14. Verwendung von 3'-Azido-3'-desoxythymidin oder einem pharmazeutisch akzeptablen Salz, Solvat, Ester oder Salz eines solchen Esters davon und von (2R,cis)-4-Amino-1-(2-hydroxymethyl-1,3-oxathiolan-5-yl)-(1H)-pyrimidin-2-on oder einem pharmazeutisch akzeptablen Salz, Solvat, Ester oder Salz eines solchen Esters davon zur Herstellung einer pharmazeutischen Zusammensetzung gemäß einem der Ansprüche 1 bis 13 zur Behandlung oder Prävention einer HIV-Infektion in einem Säuger

## Revendications

1. Composition pharmaceutique comprenant :
i) une formulation à libération contrôlée de 3'-azido-3'-désoxythymidine ou d'un quelconque sel, solvate, ester ou sel d'un tel ester acceptable du point de vue pharmaceutique de celle-ci ; et
ii) une formulation à libération immédiate de 3'-azido-3'-désoxythymidine ou d'un quelconque sel, solvate, ester ou sel d'un tel ester acceptable du point de vue pharmaceutique de celle-ci et de (2R,cis)-4-amino-1-(2-hydroxyméthyl-1,3-oxathiolan-5-yl)-(1H)-pyrimidin-2-one ou d'un quelconque sel, solvate, ester ou sel d'un tel ester acceptable du point de vue pharmaceutique de celle-ci.

2. Composition pharmaceutique comprenant :
i) une couche contenant une formulation à libération contrôlée de 3'-azido-3'-désoxythymidine ou d'un quelconque sel, solvate, ester ou sel d'un tel ester acceptable du point de vue pharmaceutique de celle-ci ; et
ii) une seconde couche contenant une formulation à libération immédiate de (2R,cis)-4-amino-1-(2-hydroxyméthyl-1,3-oxathiolan-5-yl)-(1H)-pyrimidin-2-one ou d'un quelconque sel, solvate, ester ou sel d'un tel ester acceptable du point de vue pharmaceutique de celle-ci et de 3'-azido-3'-désoxythymidine ou d'un quelconque sel, solvate, ester ou sel d'un tel ester acceptable du point de vue pharmaceutique de celle-ci.

3. Composition pharmaceutique selon la revendication 1 ou 2, dans laquelle la formulation à libération contrôlée libère la 3'-azido-3'-désoxythymidine en 3 à 6 heures.

4. Composition pharmaceutique selon l'une quelconque des revendications 1 à 3, dans laquelle la formulation à libération contrôlée comprend un mélange de polymères.

5. Composition pharmaceutique selon la revendication 4, dans laquelle les polymères sont une hydroxypropylméthyl cellulose.

6. Composition pharmaceutique selon l'une quelconque des revendications 1 à 5, dans laquelle la quantité de (2R,cis)-4-amino-1-(2-hydroxyméthyl-1,3-oxathiolan-5-yl)-(1H)-pyrimidin-2-one est de 150 à 450 mg par forme d'administration unitaire.

7. Composition pharmaceutique selon l'une quelconque des revendications 1 à 5, dans laquelle la quantité de 3'-azido-3'-désoxythymidine est de 100 à 750 mg par forme d'administration unitaire.

8. Composition pharmaceutique selon l'une quelconque des revendications 1 à 6, dans laquelle la quantité de (2R,cis)-4-amino-1-(2-hydroxyméthyl-1,3-oxathiolan-5-yl)-(1H)-pyrimidin-2-one est fournie sous une forme substantiellement dépourvue du (+)-énantiomère correspondant.

9. Composition pharmaceutique selon l'une quelconque des revendications 1 à 6, dans laquelle la (2R,cis)-4-amino-1-(2-hydroxyméthyl-1,3-oxathiolan-5-yl)-(1H)-pyrimidin-2-one est fournie de telle sorte que le (+)-énantiomère correspondant est présent en une quantité qui ne dépasse pas environ 5% en poids/poids de la quantité de (2R,cis)-4-amino-1-(2-hydroxyméthyl-1,3-oxathiolan-5-yl)-(1H)-pyrimidin-2-one.

10. Composition pharmaceutique selon l'une quelconque des revendications 1 à 9, pour une administration quotidienne unique.

11. Composition pharmaceutique selon l'une quelconque des revendications 1 à 9 sous la forme d'un comprimé bicouche ou d'un comprimé multicouche.

12. Composition pharmaceutique selon l'une quelconque des revendications 1 à 9 sous la forme d'un comprimé bicouche.

13. Composition pharmaceutique selon l'une quelconque des revendications 1 à 9 sous la forme d'un comprimé à l'intérieur d'un comprimé.

14. Utilisation de 3'-azido-3'-désoxythymidine ou d'un quelconque sel, solvate, ester ou sel d'un tel ester acceptable du point de vue pharmaceutique de celle-ci et de (2R,cis)-4-amino-1-(2-hydroxyméthyl-1,3-oxathiolan-5-yl)-(1H)-pyrimidin-2-one ou d'un quelconque sel, solvate, ester ou sel d'un tel ester acceptable du point de vue pharmaceutique de celle-ci, pour la préparation d'une composition pharmaceutique selon l'une quelconque des revendications 1 à 13 pour le traitement ou la prévention d'un infection à VIH chez un mammifère.
